Europäisches Patentamt

European Patent Office

Office européen des brevets

(11) Veröffentlichungsnummer: **0 220 545**
**B1**

(12) **EUROPÄISCHE PATENTSCHRIFT**

(45) Veröffentlichungstag der Patentschrift: **04.04.90**

(21) Anmeldenummer: **86113778.4**

(22) Anmeldetag: **04.10.86**

(51) Int. Cl.⁵: **B 01 D 61/14, G 01 N 33/24**

(54) **Elektro-Ultrafiltrationsverfahren, insbesondere zur Bodenuntersuchung.**

(30) Priorität: **22.10.85 DE 3537467**

(43) Veröffentlichungstag der Anmeldung:
**06.05.87 Patentblatt 87/19**

(45) Bekanntmachung des Hinweises auf die
Patenterteilung:
**04.04.90 Patentblatt 90/14**

(84) Benannte Vertragsstaaten:
**AT BE CH DE FR GB IT LI NL**

(56) Entgegenhaltungen:
**DE-A-2 103 111**
**US-A-3 430 486**

**PLANT AND SOIL, Band 83, 1985, Seiten 1-19,
Martinus Nijhoff/Dr.W. Junk Publishers,
Dordrecht, NL; K. NEMETH: "Recent advances in
EUF research (1980-1983)"**

(73) Patentinhaber: **Kálmán, Németh Dr.**
**Berliner Strasse 22**
**D-8703 Ochsenfurt (DE)**

(72) Erfinder: **Kálmán, Németh Dr.**
**Berliner Strasse 22**
**D-8703 Ochsenfurt (DE)**

## Beschreibung

Die Erfindung betrifft ein Elektro-Ultrafiltrationsverfahren nach dem Oberbegriff des Anspruchs 1. Solche Elektro-Ultrafiltrationsverfahren zur Bestimmung der Nährstofffraktionen im Boden sind bekannt aus der DE—C—2103111 sowie aus folgenden Veröffentlichungen: Németh, K., 1976: "Die effektive und potentielle Nährstoffverfügbarkeit im Boden und ihre Bestimmung mit Elektro-Ultrafiltration (EUF)", Habilitationsschrift, Univer-sität Gießen; Németh, K., 1985: "Recent advances in EUF research (1980—1983)", Plant and Soil 83, 1—19. Dieses dynamische Verfahren hat sich bei der Untersuchung der Nährstoffversorgung land- und forstwirtschaftlich genutzter Standorte weltweit bewährt.

Bei diesem Verfahren wird ein Elektro-Ultrafiltergerät mit variabler Spannung und Temperatur zur Herauslösung der Bodennährstoffe benutzt. Die Ultrafiltration erfolgt bei diesen Geräten mit semipermeablen Filtern, indem an Anode und Kathode ein Vakuum angelegt wird, und zwar meistens anodisch −0,7 bar und kathodisch −0,5 bar. Da diese Ultrafilter nicht mit konstanter Wasserdurchlässigkeit hergestellt werden können, schwanken die bei gegebenem Vakuum in der Zeiteinheit durchgeflossenen Filtratmengen sehr stark. Wenn aber zu Beginn der Extraktion zu große Filtratmengen durch die Anode fließen, werden die negativ geladenen Tonminerale des Bodens (Nährstoffträger des Bodens) zu schnell zur Anode transportiert, und zwar zusammen mit ihren Nährstoffen, wodurch zu wenig Nährstoffe extrahiert werden. Die Nährstoffversorgung des betreffenden Bodens wird dadurch unterbewertet, was zur Empfehlung zu hoher Düngermengen führt.

Versucht man das Vakuum nach Erreichen bestimmter Filtratmengen abzuschalten, um ein zu schnelles Mitschleppen der negativ geladenen Bodenkolloide zu verhindern und eine zu starke Verdünnung der Filtrate zu vermeiden, kommt es zu Reaktionsstörungen an den Elektroden. Wird nähmlich das Vakuum zeitweise abgeschaltet und damit die Filtration unterbrochen, werden die herausgelösten Nährstoffe nicht von den Elektroden entfernt. Eine Fraktionierung der Nährstoffe in der Zeiteinheit ist dann nicht mehr gegeben, sondern es kommt zur Verschleppung der Nährstoffe von einer Fraktion in die andere.

Der Erfindung liegt die Aufgabe zugrunde, ein zu schnelles Mitschleppen der negativ geladenen Bodenkolloide zur Anode infolge zu hoher Filtratmengen zu Beginn der Extraktion zu verhindern und eine zu starke Verdünnung der Filtrate zu vermeiden. Diese Aufgabe wird erfindungsgemäß dadurch gelöst, daß die anodische Vakuumleistung von Beginn der Extraktion an von −0,4 bar kontinuierlich bis auf −0,8 bar erhöht wird. Weitere vorteilhafte Ausgestaltungen der Erfindung sind in den Unteransprüchen angegeben.

Die mit dem Verfahren erzielten Vorteile bestehen insbesondere darin, daß zu Beginn der Extraktion, wenn die Durchlässigkeit des Anodenfilters durch die Bodenkolloide noch nicht herabgesetzt ist, eine Vakuumleistung von −0,4 bar bei den verschiedensten Böden mit unterschiedlichsten Kolloidgehalten genügt, um ausreichende Filtratmengen zu gewinnen. Bei einer Vakuumleistung von nur −0,4 bar können auch bei niedrigen Kolloidgehalten keine großen Filtratmengen auftreten, so daß ein Mitschleppen der Bodenkolloide zur Anode vermieden wird. Während der Extraktion wird die Durchlässigkeit des Anodenfilters durch die Anlegerung von Ionmineralien herabge-setzt. Mit der kontinuierlich steigenden Vakuum leistung von −0,4 bis −0,8 bar wird der Herabsetzung der Durchlässigkeit des Anodenfilters entgegengewirkt, und es können ausreichende anodische Filtratmengen gewonnen werden.

Mit Hilfe der vorgeschlagenen kontinuierlich steigenden Vakuum leistung kann also die Näh-stoffversorgung der Böden genauer beurteilt werden, als das bisher möglich war, wodurch die Düngeberatung genauer erfolgen kann. Eine dem Bedarf der Pflanze angepaßte Düngung bietet die Voraussetzung für die Erzeugung qualitativ hochwertiger Nahrungsmittel und dient zugleich der Umweltsicherung.

## Patentansprüche

1. Elektro-Ultrafiltrationsverfahren zur Untersu-chung von Nähr- oder Schadstoffen, insbesondere in Böden, Müllkompost oder Klärschlämmen, bei dem die an die Elektroden angelegte elektrische Spannung während der Extraktion variiert wird und bei dem die durch die Ultrafilter zu den Elektroden transportierten Stoffe durch Unter-druck abgesaugt werden, dadurch gekennzeich-net, daß die anodische Vakuumleistung von Beginn der Extraktion an von −0,4 bar kontinuierlich bis auf −0,8 bar erhöht wird.

2. Elektro-Ultrafiltrationsverfahren nach Anspruch 1, dadurch gekennzeichnet, daß die kathodische Vakuumleistung von Beginn der Extraktion an von −0,4 bar bis auf −0,6 bar kontinuierlich erhöht wird.

3. Elektro-Ultrafiltrationsverfahren nach Anspruch 1 und 2, dadurch gekennzeichnet, daß die Anfangs- und Endwerte der anodischen und kathodischen Vakuen je nach Durchlässigkeit der verwendeten Ultrafilter erhöht oder erniedrigt werden.

## Revendications

1. Procédé d'électro-ultrafiltration pour l'analyse de substances nutritifs ou toxiques, surtout dans des sols, du compost à base d'ordures ou boues de curage, la tension électrique appliquée au niveau des électrodes variant pendant l'extraction et les substances transportées vers les électrodes par les ultrafiltres étant aspirées par sous-pression, le procédé étant caractérisé par l'augmentation continue de la puissance de vide anodique, de −0,4 bar au début de l'extraction jusqu'à une valeur de −0,8 bar.

2. Procédé d'électro-ultrafiltration comme celui de la position 1, mais caractérisé par l'augmentation continue de la puissance de vide cathodique, de −0,4 bar au début de l'extraction jusqu'à une valeur de −0,6 bar.

3. Procédé d'électro-ultrafiltration comme celui des positions 1 et 2, caractérisé par le fait que les valeurs initiale et finale des vides anodiques et cathodiques sont augmentées ou réduites suivant la perméabilité des ultrafiltres utilisés.

**Claims**

1. Electro-ultrafiltration process for the examination of nutrients or pollutants, particularly in soil, refuse compost or sewage sludges. In this process, the electrical voltage applied to the electrodes is varied during extraction and the substances transported through the ultrafilters to the electrodes are extracted by vacuum, the anodic vacuum being continually increased from −0.4 bar to −0.8 bar upon commencement of extraction.

2. Electro-ultrafiltration process as described in claim 1, with the cathodic vacuum being continually increased from −0.4 bar to −0.6 bar upon commencement of extraction.

3. Electro-ultrafiltration process as described in claims 1 and 2, with the initial and final anodic and cathodic vacuums being increased or reduced in relation to the permeability of the ultrafilters used.